# EUROPÄISCHE PATENTSCHRIFT

(11) **EP 1 287 118 B1**
(45) Veröffentlichungstag und Bekanntmachung des Hinweises auf die Patenterteilung: **16.01.2008**
(21) Anmeldenummer: 01943147.7
(22) Anmeldetag: 28.05.2001
(51) Int. Cl.: C12N 5/06, C12N 5/08, C12N 5/10, A61L 27/38

(54) **VERFAHREN ZUR HERSTELLUNG EINES EMPFÄNGERSPEZIFISCHEN GEWEBE-TRANSPLANTATS ODER -IMPLANTATS**
METHOD FOR PRODUCING A RECIPIENT-SPECIFIC TISSUE TRANSPLANT OR TISSUE IMPLANT
PROCEDE POUR FABRIQUER UN TRANSPLANT OU UN IMPLANT DE TISSU SPECIFIQUE A UN RECEVEUR

(30) Priorität: 29.05.2000 DE 10026480
(43) Veröffentlichungstag der Anmeldung: 05.03.2003
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen
(86) Internationale Anmeldenummer: PCT/DE2001/001984
(87) Internationale Veröffentlichungsnummer: WO 2001/092475

(56) Entgegenhaltungen:
- WO-A-95/24873
- WO-A-99/00152
- WO-A-99/52572

## Beschreibung

Die Erfindung betrifft ein verfahren zur Herstellung eines empfängerspezifischen Gewebe-Transplantats oder Gewebe-Implantats aus einer Gewebematrix und darauf angesiedelten empfängerverträglichen Zellen.

In der Transplantationsmedizin besteht ein großes Bedürfnis nach geeigneten, in möglichst geringem Maße adverse Reaktionen des Transplantat-Empfängers auslösenden Transplantaten. Nur in bestimmten Fällen ist es möglich, das Transplantat aus dem Körper des Empfängers selbst zu entnehmen und zu verpflanzen. Aus immunologischer Sicht, sind diese Transplantationen am unbedenklichsten, bei bestimmten Gefäßen oder Organen sowie bei größeren zu ersetzenden Hautbereichen besteht diese Möglichkeit jedoch nicht. Für bestimmte Organe kommen heute praktisch nur allogene Transplantate fremder Spender oder - vielfach im orthopädischen Bereich - synthetische Implantate aus Kunststoffen, Metallen, Keramik usw. bzw. verschiedenen Verbundstoffen in Betracht. Bei der Verwendung allogener Materialien, wie z.B. Spenderorganen ist eine ständige für den Organismus des Empfängers belastende Immunsuppression nötig. Dennoch kommt es häufig zu Abstoßungsreaktionen als ernsten Komplikationen. Auch künstliche Materialien können zu Abstoßungsreaktionen und entzündlichen Prozessen führen, die das Operationsergebnis zunichte machen.

Aus verschiedenen Gründen wird heute oft versucht, xenogenes Material (tierischen Ursprungs) zu verwenden. Vorteilhaft ist hierbei vor allem die bessere Verfügbarkeit dieses Materials gegenüber allogenen (Spender-)Materialien. Auch ist ein solches "biologisches Material" flexibler als ein künstliches Material und paßt sich an manchen Stellen dem Empfängerkörper besser an. Das xenogene Transplantationsmaterial ist jedoch deshalb problematisch, da es stark antigen ist.

Es wird daher seit längerer Zeit versucht, xenogene allogene oder synthetische Transplantationsmaterialien, d.h. verschiedenartige für eine Transplantation vorgesehene Gewebe, empfängerverträglich zu machen. Hierzu wird häufig versucht, durch Azellularisierung immunologisch für den Empfänger weitgehend neutralisierte xenogene oder allogene Gewebe oder synthetische Matrix-Materialien (beispielsweise aus biologisch abbaubaren Biopolymeren) mit empfängerverträglichen Zellen zu besiedeln, um so zu einem Transplantat oder Implantat zu kommen, das vom Empfänger möglichst nicht als körperfremd erkannt oder zumindest wegen der vorhandenen autologen, körpereigenen Zellen besser toleriert wird.

In der DE 19828726 A1 wird beispielsweise ein Verfahren zur Herstellung eines bioartifiziellen Transplantats beschrieben, bei dem zunächst native Zellen von dem interstitiellen Bindegewebe des Transplantats entfernt werden. Die Matrix wird danach mit für den Empfänger verträglichen, vorzugsweise autologen Zellen neu besiedelt, so dass ein empfängerspezifisches Biotransplantat erhalten wird.

Um das naturähnliche Anwachsen der für die Neu-Besiedlung verwendeten Zellen und die anhaltende ständige Zellerneuerung zu fördern, ist es vorteilhaft, wenn der Kultur bestimmte zelluläre Mediatoren oder auch Faktoren zugeführt werden, die die anwachsenden Zellen zu bestimmten Prozessen stimulieren. Umfassen die aufgebrachten empfängerspezifischen Zellen Fibroblasten, so ist es z.B. mit Hilfe geeigneter Mediatoren/Faktoren möglich, die Kollagenneubildung anzuregen und so die Umbildung der Gewebematrix in eine für den Empfänger autologe zu unterstützen. Zahlreiche Faktoren wirken chemotaktisch oder wachstumsbeschleunigend, wobei dem Fachmann die Wirkungen der einzelnen Faktoren grundsätzlich soweit untersucht bekannt sind. Durch die Zugabe der Faktoren werden die genannten Prozesse ausgelöst, beschleunigt und durch die Art und Funktion der Faktoren gesteuert.

Bei der im Stand der Technik bekannten Vorgehensweise besteht der Nachteil, dass die verwendbaren Faktoren sehr teuer sind. Dies beschränkt die Einsatzbreite der vorgenannten Verfahren, da die Herstellung empfängerspezifischer Transplantate oder Implantate durch Besiedeln bestimmter Transplantat-Grundkörper mit empfängerverträglichen Zellen bereits sehr teuer ist und sich das Verfahren durch den Einsatz natürlicher isolierter oder synthetisierter Mediatoren noch weiter verteuert.

Ein weiteres Problem besteht in der genauen Dosierung der Mediatoren/Faktoren und Co-Faktoren und der Steuerung ihres zeitlich wie örtlich optimalen Einsatzes

Die Aufgabe der Erfindung besteht daher darin, bei einem Verfahren zur Herstellung eines empfängerspezifischen Gewebe-Transplantats oder Gewebe-Implantats aus einer Gewebematrix und darauf angesiedelten empfängerverträglichen Zellen die Zuführung von Mediatoren, Faktoren, Co-Faktoren zu der Gewebekultur zu vereinfachen, zu verbilligen und besser zu steuern. Gleichzeitig soll das Verfahren eine noch natürlichere Ausbildung des erhaltenen empfängerspezifischen Transplantats oder Implantats ermöglichen.

Zur Lösung dieses Problems ist erfindungsgemäß vorgesehen, dass bei einem Verfahren zur Herstellung eines empfängerspezifischen Gewebe-Transplantats oder Gewebe-Implantats aus einer Gewebematrix und darauf angesiedelten empfängerverträglichen Zellen, die Besiedlung der Gewebematrix mit den empfängerverträglichen Zellen unter Beteiligung zusätzlicher Zellen durchgeführt wird, die Mediatoren, Faktoren oder Co-Faktoren produzieren und in die Umgebung abgeben, die eine Geweberegeneration fördern

Unter einer "Beteiligung" Mediatoren oder Faktoren abgebender zusätzlicher Zellen soll hier verstanden werden, dass diese zusätzlich zu den für die Besiedlung verwendeten empfängerverträglichen Zellen innerhalb des Besiedlungsverfahrens entweder in die Gewebekultur direkt eingebracht werden und durch Wechselwirkung mit den empfängerspezifischen Zellen zur Abgabe der Mediatoren und Faktoren angeregt werden (so dass sie in den Besiedlungsvorgang direkt eingreifen, also beteiligt sind) oder parallel zur Kultur des Gewebe-Transplantats oder - Implantats innerhalb der selben Kultivierungsvorrichtung (Bioreaktor) oder in einer gesonderten Apparatur gleichzeitig kultiviert werden (Co-Kultur), wobei die von den erfindungsgemäß verwendeten Zellen in die Parallelkultur ausgeschiedenen Produkte (Mediatoren, Faktoren, Co-Faktoren) der Gewebekultur unmittelbar zugeführt werden. Zu den Mediatoren gehören auch entzündungsauslösende Mediatoren.

Bei einer Parallelkultur der Mediatoren oder Faktoren abgebenden Zellen kann dies in einem üblichen für die Kultur der jeweiligen Zellart geeigneten Kulturmedium geschehen.

Unter "Mediatoren oder Faktoren abgebenden Zellen" werden im Rahmen dieser Erfindung solche verstanden, die vergleichsweise gut zur Abgabe von Mediatoren, Faktoren oder Co-Faktoren, die eine Geweberegeneration fördern,aktiviert, d.h. angeregt werden können. Hierzu zählen u.a. Makrophagen, bestimmte immunkompetente Zellen und andere. Die Aktivierung kann vorzugsweise durch einen von den Besiedlungszellen ausgehenden Stimulus (z.B. Zelldetritus) erfolgen, alternativ auch durch Zugabe chemischer Stoffe oder kleiner Fremdpartikel.

Unter einer Geweberegeneration werden Reparaturprozesse sowie Ab- und Umbauprozesse des Gewebes verstanden. Die Mediatoren abgebenden Zellen stimulieren umgebende Zellen zu jeweils phänotypischen Umbildungen, fördern neosynthetische Prozesse und Gewebeerneuerung.

Als Mediatoren oder Faktoren abgebende Zellen können autologe Zellen des Transplantatempfängers verwendet werden. Insbesondere sind vorgesehen: Leukozyten, wie Lymphozyten, Makrophagen, Granulozyten, dendritische Zellen, Stammzellen, insbesondere pluripotente Stammzellen bzw. somatische Stammzellen oder Mischungen dieser Zellen. Die Verwendung von Makrophagen wird derzeit als besonders vorteilhaft betrachtet.

Insbesondere bei einer Co-Kultur der Mediatoren oder Faktoren abgebenden Zellen, jedoch auch bei direkter Zugabe in die Transplantat-Gewebekul-tur, können diese Zellen durch Zugabe geeigneter Stoffe zur Produktion bestimmter Mediatoren, Faktoren oder Co-Faktoren oder zu einer verstärkten Produktion dieser Zellprodukte angeregt werden. Zur Anregung der Zellen kann u.a. Zelldetritus aus der Gewebekultur dienen.

Vorzugsweise wird das Verfahren so geführt, dass die Mediatoren oder Faktoren abgebenden Zellen während der Besiedlung wenigstens zeitweilig einem für die Besiedlung verwendeten Kulturmedium und/oder der Gewebematrix direkt zugeführt werden.

Die Besiedlung der Gewebeunterlage mit empfängerverträglichen Zellen zu einem Gewebe-Transplantat oder -Implantat kann in an sich bekannter Weise erfolgen, z.B. wie in der DE 198 28 726 im einzelnen angegeben. Die Gewebe-Unterlage oder Gewebematrix - beispielsweise eine natürliche oder synthetische, azellularisierte oder native Kollagen-Matrix oder ein synthetisches Gewebe, wie eine Biopolymer-Faser- oder -netzstruktur - befindet sich hierbei im allgemeinen in einem Kulturmedium, dem verschiedene Zusätze beigegeben sein können (auch konditionierendes Medium genannt, gemeint ist die Konditionierung des Gewebes für die Besiedlung). Das Kulturmedium kann beispielsweise über dem Gewebe bewegt oder in einem Kreislauf über dem Gewebe umgewälzt werden. Das Kulturmedium kann auch in Intervallen zugegeben und dann eine Zeit stehend über der Kultur belassen werden; die optimalen Bedingungen richten sich u.a. nach der zu besiedelnden Gewebeart.

Als Kulturmedium bzw. konditionierendes Medium kann ein übliches Nährmedium verwendet werden, das ggf. mit verschiedenen Zusätzen versehen sein kann. Hierfür geeignete Nährmedien sind dem Fachmann bekannt.

In das Medium werden empfängerspezifische Zellen eingebracht, entweder zu Beginn der Besiedlung, kontinuierlich oder in mehreren Schüben. Unter empfängerspezifischen Zellen werden für den Empfänger autologe, für den Empfänger als immunologisch möglichst verträglich ausgewählte (kompatible) allogene oder genetisch veränderte allogene oder xenogene Zellen verstanden. Als immunologisch kompatibel können Zellen dann angesehen werden, wenn sie durch Tests als empfänger-ähnlich klassifiziert wurden oder wenn sie insbesondere durch genetische Veränderung an den Empfänger angepasst wurden.

Es können auch zu unterschiedlichen Besiedlungs- bzw. Behandlungszeitpunkten verschiedene Arten von Zellen aufgegeben werden, so dass sich auf dem Gewebe unterschiedliche Zellschichten aus verschiedenartigen Zellen aufbauen können. Weiterhin können örtlich verschiedene Zellen aufgebracht werden, beispielsweise auf der Oberseite und der Unterseite eines Hauttransplantats unterschiedliche Zellen oder auf der Innenseite und der Außenseite eines röhrenförmigen Gefäßes unterschiedliche Zellen. Als empfängerverträgliche Zellen kommen grundsätzlich alle Arten von Körperzellen in Frage, beispielsweise:

Bindegewebszellen (u.a.Fibroblasten, Fibrozyten), Muskelzellen (Myozyten), Endothelzellen, Hautzellen (u.a. Keratinozyten), zu Organzellen differenzierte Zellen (Herzzellen, Nierenzellen, usw.), vorzugsweise bei strukturierten Organen mit Kollagengerüst, allgemein alle Zellen, die für den Umbau eines bestimmten, für die Implantation vorgesehenen Gewebes sinnvollerweise angeboten werden können

Bei dem Gewebe-Transplantat kann es sich grundsätzlich um jedes transplantierbare Gewebe handeln. Insbesondere zählen hierzu: allgemein Gefäße, wie Aorten und Venen, Aortenklappen, Herzklappen, Organteile und ganze Organe, Hautstücke, Sehnen, Cornea, Knorpel, Knochen, Larynx, Herz, Trachea, Nerven, Miniskus, Diskus intervertebralis, Ureteren, Urethra, Blase, u.a.m

Implantate auf Basis synthetischer Matrizes oder Gerüste bieten sich unter vielen anderen Möglichkeiten für Venen, Herzklappen, Cornea, Blase und Haut an.

Die Mediatoren oder Faktoren abgebenden Zellen können nun, wie oben beschrieben, während der Besiedlung wenigstens zeitweilig dem Gewebe-Kulturmedium und/oder der Gewebematrix zugeführt werden. Hierfür ist es möglich, Mediatoren oder Faktoren abgebende Zellen zu Beginn der Besiedlung einmalig oder gemeinsam mit den empfängerverträglichen Zellen auf die Gewebematrix direkt aufzubringen. Die Mediatoren oder Faktoren abgebenden Zellen können auch mit dem Kulturmedium schubweise oder kontinuierlich zugeführt werden, wobei auch ein Wechsel zwischen Kulturmedium ohne und mit Mediatoren oder Faktoren abgebenden Zellen erfolgen kann.

In Weiterbildung der Erfindung ist auch vorgesehen, dass für die Zuführung der Mediatoren oder Faktoren abgebenden Zellen Blut verwendet wird, das die gewünschten Mediatoren oder Faktoren abgebenden Zellen von Natur aus enthält, vorzugsweise Eigenblut des Transplantat- oder Implantatempfängers. Dieses Blut kann bezüglich der gewünschten Bestandteile aufkonzentriert werden oder es können Blutbestandteile separiert und hier verwendet werden.

Weiter vorzugsweise können die für die Abgabe von Mediatoren oder Faktoren aktivierbaren Zellen in einer Kultur gehalten werden, die mit dem Gewebe während der Besiedlung so in Verbindung steht, dass aus der Zellkultur abgegebene Mediatoren/Faktoren dem Gewebe während der Besiedlung zugeführt werden. Wie oben beschrieben ist diese Co-Kultur Mediatoren oder Faktoren abgebender Zellen als Parallelkultur außerhalb oder innerhalb der Vorrichtung für die Gewebekultur möglich.

Die Kultur Mediatoren oder Faktoren abgebender Zellen, beispielsweise eine Makrophagen-Kultur, kann in einem Bioreaktor erfolgen, der mit dem Reaktor, in dem das empfängerspezifische Transplantat oder Implantat gezüchtet und behandelt wird, in geeigneter Weise verbunden ist. Aus dem Bioreaktor abgenommene Faktoren können dem umlaufenden oder schubweise zugeführten konditionierenden Kulturmedium in geeigneter Menge zugeführt werden.

Alternativ kann die Makrophagen-Kultur, bzw. die Kultur sonstiger Mediatoren oder Faktoren abgebender Zellen oder Mischung Mediatoren oder Faktoren abgebender Zellen, während der Schritte der Behandlung mit empfängerverträglichen Zellen durch ein für die zellulären Mediatoren und/oder Faktoren durchlässige Folie, Membran oder Trennwand von dem konditionierenden Medium getrennt gehalten werden, sodass die gebildeten Mediatoren und/oder Faktoren kontinuierlich in das konditionierende Medium abgegeben werden können.

Die Behandlung des für die Transplantation vorgesehenen Gewebes wird im allgemeinen in einem Bioreaktor erfolgen, in dem das Kulturmedium innerhalb eines bestimmten Raumes vorgehalten und ggf. umgewälzt wird. Innerhalb dieses Raumes kann mit einer meablen Trennwand ein Kulturraum für die Kultur der erfindungsgemäß verwendeten Zellen oder Makrophagen ausgebildet werden, so dass die gebildeten zellulären Mediatoren und/oder Faktoren ständig in das konditionierende Medium auswandern können.

Als Mediatoren oder Faktoren abgebende Zellen können insbesondere verwendet werden: Zellen aus der Familie der Leukozyten , jedoch auch periphere oder zentrale Stammzellen (aus Blut, Fettgewebe, Organen und Knochenmark), vorzugsweise pluripotente Stammzellen, so z.B. alle Formen der weißen Blutkörperchen, Granulozyten, Lymphozyten, Makrophagen, Monozyten, Knochenmarkszellen, Milzzellen, Memory-Zellen, Thymuszellen.

Für die Besiedlung mit Hilfe von empfängerverträglichen Zellen werden jeweils Zellen oder Gemische von Zellen ausgewählt, die zu dem jeweiligen Gewebetyp passen. Die empfängerverträglichen, vorzugsweise autologen oder genetisch modifizierten und hierdurch empfängerspezifisch gemachten allogenen oder xenogenen Zellen umfassen solche Zellen, die für den Aufbau des gewünschten Gewebes geeignet sind, und wahlweise zusätzlich solche, die die Gewebeumbildung mit stimulieren und/oder kontrolieren können, wie z.B. zelluläre Faktoren produzierende Zellen und/oder chemotaktisch beeinflussende Zellen, hierunter die oben genannten Mediatoren oder Faktoren abgebenden Zellen.

Ein Vorteil der Erfindung, d.h. der Beteiligungvon Zellen, die Mediatoren, Faktoren, Co-Faktoren prodzuzieren und an das konditionierende Kultur-Medium abgeben, bei der Kultur eines mit empfängerverträglichen Zellen besiedelten Transplantats oder Implantats liegt darin, dass für den jeweiligen Zweck besonders geeignete Mediatoren/Faktoren während eines ohnehin erforderlichen Kulturschritts mitproduziert werden können, so dass auf die Verwendung zusätzlicher teurer und weniger spezifischer Faktoren verzichtet werden kann.

Durch die Vorgabe bestimmter zur Abgabe von Mediatoren oder Faktoren aktivierbarer Zellen und ggf. geeignete Anregung dieser Zellen kann die Abgabe der Mediatoren/Faktoren beeinflusst und so der Gewebeaufbau gesteuert sowie beschleunigt werden.

Als Gewebematrix kann - je nach Anwendungsfall - beispielsweise eine synthetische Gewebematrix verwendet werden, die z.B. eines oder mehrere der folgenden Materialien umfassen kann: Polyglactid, Polydioxanon, biologisch abbaubare Polyester, Polyurethane, Polyacryle, Kollagen, Fibrinogen. Alternativ können, wie oben bereits beschrieben, auch native oder azellularisierte xenogene oder allogene Gewebematrices verwendet werden.

Im folgenden wird die Erfindung anhand von Beispielen beschrieben:

**Beschleunigter Umbau von technischen und biologischen Polymeren als Trägersubstanzen (Gewebe-Matrix) für neue bioartifizielle Gewebe.**

Es werden biologisch abbaubare Polymere als synthetische Matrixmaterialien verwendet. Bei den synthetischen Materialien kann es sich auch um aufgereinigte Materialien biologischen Ursprungs handeln. Diese Materialien werden zu "synthetischen" Matrizen (im Vergleich zu azellularisierten biologischen Geweben) geformt.

Der Polymerabbau kann hydrolytisch oder auch enzymatisch erfolgen.

Eingesetzt werden können biodegradable Polymere wie Polyglactid, Polydioxanon, Polyester, Polyurethane, Polyacryle, spezielle biologische Polymere und (Bio)-Makromeleküle, wie Kollagen oder Fibrinogen, die klassischen Bestandteile extrazellulärer Matrix.

Entsprechend einem üblichen Verfahren wird eine synthetische Gewebeunterlage (beispielsweise eine synthetische Herzklappe) mit den ausgewählten empfängerspezifischen Zellen besiedelt. Die Kultur erfolgt in einem hierfür üblichen Medium (z.B. DMBM; WE). Während der Besiedlung wird das Gewebe-Implantat zeitweise in Intervallen mit Eigenblut des ImplantatEmpfängers, aufkonzentriertem Eigenblut oder mit Blutbestandteilen versetztem Kulturmedium durchspült. In dieser Phase adhärieren Makrophagen selektiv an der exponierten Matrix. Lymphozyten und Makrophagen erhalten immunstimulatorische Reize durch die Spaltprodukte der Polymere und werden aktiviert, die autologen Myofibroblasten über endogene Aktivatoren anzuregen Matrix zu synthetisieren. Dies ist besonders wichtig, da dann der z.B. hydrolytische Abbau der Polymere beschleunigt erfolgen kann, sodass dieser erheblich kürzer ist als bisher möglich. Dies ermöglicht wiederum die Implantation eines fertigeren Produkts in vivo ohne vorhandene Restpolymere, die in vivo ungewünschte Instabilitäten bei plötzlichem akzelerierten Zerfall oder auch Fremdkörpereaktionen auslösen können.

Alternativ zu einer Kultur in Blut können Präparationen von Blutplättchen (Gewinnung bei ca. 3000 g und weiße Blutkörperchen 1800 g) separat in unterschiedlichen Bereichen des Bioreaktors oder synchron in einer gesonderten Apparatur kokultiviert werden. In letzterem Falle werden die so erhaltenen Kulturprodukte der Gewebekultur in dem eigentlichen Gewebe-Bioreaktor zugeführt.

### Induktion eines Remodelling in Herzklappen zu einem normalen zellphysiologischen Profil:

Die Vorexpansionsphase der vaskulären Zellen des Empfängers benötigt bei herkömmlichen Verfahren circa 10 Tage. Herzklappen werden danach in einem Bioreaktor eingebracht und unter Verwendung der vorexpandierten Fibroblasten, glatten Muskelzellen (SMC) und Endothelialen wie üblich luminal besiedelt. Parallel dazu werden frisch gewonnene Knochenmarksstammzellen als Gesamtpool auf und in die Matrix mittels einer Spritze aufgebracht. Dort findet unter dem lokalen Mikroumgebungsdruck der räumlichen Position extrazellulärer Matrix eine Differenzierungssteuerung der Stammzellen statt. Der Aktivierungszustand der Stammzellen wird zusätzlich durch Matrix- und lokale Zellzerfallsprodukte angeregt. Durch die Verwendung vaskulärer Endothelzellen und glatter Muskelzellen des Empfängers findet eine wechselseitige Beeinflussung mit den Stammzellen statt. Weiterhin werden die gewebespezifischen Zellen vor Ort dadurch generiert. Dies bedeutet, dass auf Grund der Biopsieentnahmen zum Beispiel bei der Erstellung von Herzklappen der Aorta Gewebe verwendet werden muss, das nicht direkt aus dem Zielgewebe gewinnbar ist. Dies beinhaltet zum Beispiel Venenmaterial, das eine andere molekulare und phänotypische Differenzierung als arterielle Zellen aus der Aortaklappe besitzt. Weiterhin fehlen verschiedene Zelltypen, die zum Beispiel nur im Klappensegel vorkommen, wie zum Beispiel neuronale Zellsysteme.

Die Stammzellen differenzieren unter dem lokalen Mikroumgebungsdruck den in der Klappe vorhandenen Matrixparameter (idealerweise einer zum Beispiel allogenen Aortenklappenmatrix) in ortsspezifische Zelltypen einschließlich neuronaler Zellsysteme, die für lokale Innervationsprozesse verantwortlich sind. Nach der in vivo Implantation können die Stammzellen auch noch die Repopulation mit den arteriellen Zellen ermöglichen. Die EC sind aber dennoch wichtig, da im Gefäßsystem die Thrombogenizität reduziert wird. Durch die FB und SMC, selbst venösen Ursprungs, wird zumindest eine Initiation des Remodellingprozesses in empfängerspezifische Matrix erreicht, die durch die aus den Stammzellen rekrutierten Zellpools weitergeführt, korrigiert und zu Ende geführt wird.

## Patentansprüche

1. Verfahren zur Herstellung eines empfängerspezifischen Gewebe-Transplantats oder Gewebe-Implantats aus einer Gewebematrix und darauf angesiedelten empfängerverträglichen Zellen,
**dadurch gekennzeichnet ,**
**dass** die Besiedlung der Gewebematrix mit den empfängerverträglichen Zellen unter Beteiligung zusätzlicher Zellen durchgeführt wird, die Mediatoren, Faktoren oder Co-Faktoren produzieren und in die Umgebung abgeben, die eine Geweberegeneration fördern

2. Verfahren nach Anspruch 1, **dadurch gekennzeichnet, dass** die Zellen entzündungsauslösende Mediatoren oder Faktoren abgeben.

3. Verfahren nach Anspruch 1 oder 2, **dadurch gekennzeichnet, dass** die Mediatoren oder Faktoren abgebenden Zellen autologe Zellen des Transplantatempfängers, insbesondere Leukozyten, dendritische Zellen, Makrophagen oder Mischungen aus diesen Zellen umfassen.

4. Verfahren nach einem der Ansprüche 1 bis 3, **dadurch gekennzeichnet, dass** die Mediatoren oder Faktoren abgebenden Zellen vorgängerzellen oder Stammzellen umfassen.

5. Verfahren nach einem der Ansprüche 1 bis 4, **dadurch gekennzeichnet, dass** die Mediatoren oder Faktoren abgebenden Zellen während der Besiedlung wenigstens zeitweilig einem für die Besiedlung verwendeten Kulturmedium und/oder der Gewebematrix zugeführt werden.

6. Verfahren nach Anspruch 5, **dadurch gekennzeichnet, dass** die Zuführung durch Zusatz von Eigenblut oder Eigenblut-Bestandteilen des Transplantat- oder Implantatempfängers erfolgt.

7. Verfahren nach Anspruch 5 oder 6, **dadurch gekennzeichnet, dass** die Mediatoren oder Faktoren abgebenden Zellen in einer Kultur gehalten werden, die mit dem Gewebe während der Besiedlung in der Form in Verbindung steht, dass von der Kultur der Zellen abgegebene Mediatoren/Faktoren dem Gewebe während der Besiedlung zugeführt werden.

8. Verfahren nach Anspruch 7, **dadurch gekennzeichnet, dass** die Kultur der Mediatoren oder Faktoren abgebenden Zellen, vorzugsweise eine Makrophagenkultur, während der Besiedlung oder Behandlung mit empfängerverträglichen Zellen durch eine für die zellulären Mediatoren und/oder Faktoren durchlässige Folie, Membran oder Trennwand von dem Kulturmedium getrennt gehalten werden und die gebildeten Mediatoren und/oder Faktoren kontinuierlich in das Gewebe-Transplantat-Kulturmedium abgegeben werden.

9. Verfahren nach einem der Ansprüche 1 bis 8, **dadurch gekennzeichnet, dass** als Gewebematrix eine synthetische Gewebematrix verwendet wird.

10. Verfahren nach Anspruch 9, **dadurch gekennzeichnet, dass** die verwendete Gewebematrix eines oder mehrere der folgenden Materialien umfasst: Polyglactid, Polydioxanon, biologisch abbaubare Polyester, Polyurethane, Polyacryle, Kollagen, Fibrinogen.

11. Verfahren nach einem der Ansprüche 1 bis 8, **dadurch gekennzeichnet, dass** als Gewebematrix eine native oder azellularisierte xenogene oder allogene Gewebematrix verwendet wird.

12. Verfahren nach einem der Ansprüche 1 bis 11, **dadurch gekennzeichnet, dass** die verwendeten empfängerverträglichen Zellen autologe Zellen des Transplantat- oder ImplantatEmpfängers sind.

13. Verfahren nach einem der Ansprüche 1 bis 11, **dadurch gekennzeichnet, dass** die verwendeten empfängerverträglichen Zellen für den Empfänger als verträglich ausgewählte allogene oder genetisch veränderte allogene Zellen sind.

## Claims

1. Process for the production of a recipient-specific tissue graft or tissue implant comprising a tissue matrix and recipient-tolerated cells which have colonized the latter,
**characterized in that**
the colonization of the tissue matrix with the recipient-tolerated cells is carried out with the participation of additional cells which produce and release into the environment mediators, factors or cofactors which promote tissue regeneration.

2. Process according to Claim 1, **characterized in that** the cells release inflammation-initiating mediators or factors.

3. Process according to Claim 1 or 2, **characterized in that** the cells which release mediators or factors include autologous cells from the graft recipient, in particular leukocytes, dendritic cells, macrophages or mixtures of these cells.

4. Process according to one of Claims 1 to 3, **characterized in that** the cells which release mediators or factors include precursor cells or stem cells.

5. Process according to one of Claims 1 to 4, **characterized in that** the cells which release mediators or factors are fed at least temporarily during the colonization to a culture medium which is used for the colonization and/or to the tissue matrix.

6. Process according to Claim 5, **characterized in that** the feed is carried out by adding the graft or implant recipient's own blood or blood constituents.

7. Process according to Claim 5 or 6, **characterized in that** the cells which release mediators or factors are kept in a culture which is connected to the tissue during the colonization in such a way that mediators/ factors released by the cell culture are fed to the tissue during the colonization.

8. Process according to Claim 7, **characterized in that** the culture of the cells which release mediators or factors, preferably a macrophage culture, is kept separate from the culture medium during the colonization or treatment with recipient-tolerated cells by means of a film, membrane or partition which is permeable to the cellular mediators and/or factors, and the mediators and/or factors formed are released continuously into the tissue graft culture medium.

9. Process according to one of Claims 1 to 8, **characterized in that** the tissue matrix used is a synthetic tissue matrix.

10. Process according to Claim 9, **characterized in that** the tissue matrix used comprises one or more of the following materials: polyglactide, polydioxanone, biodegradable polyesters, polyurethanes, polyacrylates, collagen, fibrinogen.

11. Process according to one of Claims 1 to 8, **characterized in that** the tissue matrix used is a native or acellularized xenogenic or allogenic tissue matrix.

12. Process according to one of Claims 1 to 11, **characterized in that** the recipient-tolerated cells used are autologous cells from the graft or implant recipient.

13. Process according to one of Claims 1 to 11, **characterized in that** the recipient-tolerated cells used are allogenic cells or genetically modified allogenic cells selected as being tolerated by the recipient.

## Revendications

1. Procédé pour la production d'une greffe tissulaire ou d'un implant tissulaire spécifique d'un receveur comprenant une matrice tissulaire et des cellules tolérées par le receveur qui ont colonisé cette dernière,
**caractérisé en ce que**
la colonisation de la matrice tissulaire à l'aide des cellules tolérées par le receveur est mise en oeuvre avec la participation de cellules additionnelles qui produisent et libèrent dans l'environnement des médiateurs, des facteurs ou des cofacteurs qui favorisent une régénération tissulaire.

2. Procédé selon la revendication 1, **caractérisé en ce que** les cellules libèrent des médiateurs ou des facteurs d'initiation d'inflammation.

3. Procédé selon la revendication 1 ou 2, **caractérisé en ce que** les cellules qui libèrent des médiateurs ou des facteurs incluent des cellules autologues issues du receveur greffé, en particulier des leucocytes, des cellules dendritiques, des macrophages ou des mélanges de ces cellules.

4. Procédé selon l'une des revendications 1 à 3, **caractérisé en ce que** les cellules qui libèrent des médiateurs ou des facteurs incluent des cellules précurseuses ou des cellules souche.

5. Procédé selon l'une des revendications 1 à 4, **caractérisé en ce que** les cellules qui libèrent des médiateurs ou des facteurs sont alimentées au moins temporairement pendant la colonisation sur un milieu de culture qui est utilisé pour la colonisation et/ou sur la matrice tissulaire.

6. Procédé selon la revendications 5, **caractérisé en ce que** l'alimentation est mise en oeuvre en ajoutant le sang propre ou des constituants du sang propre du receveur greffé ou implanté.

7. Procédé selon la revendication 5 ou 6, **caractérisé en ce que** les cellules qui libèrent des médiateurs ou des facteurs sont conservées dans une culture qui est connectée au tissu pendant la colonisation de telle sorte que des médiateurs/facteurs libérés par la culture de cellules soient alimentés sur le tissu pendant la colonisation.

8. Procédé selon la revendications 7, **caractérisé en ce que** la culture des cellules qui libèrent des médiateurs ou des facteurs, de préférence une culture macrophage, est maintenue séparée du milieu de culture pendant la colonisation ou le traitement à l'aide de cellules tolérées par le receveur au moyen d'un film, d'une membrane ou d'une séparation qui est perméable aux médiateurs et/ou facteurs cellulaires, et les médiateurs et/ou facteurs formés sont libérés en continu dans le milieu de culture de greffe tissulaire.

9. Procédé selon l'une des revendications 1 à 8, **caractérisé en ce que** la matrice tissulaire utilisée est une matrice tissulaire synthétique.

10. Procédé selon la revendications 9, **caractérisé en ce que** la matrice tissulaire utilisée comprend un ou plusieurs des matériaux qui suivent : polyglactide, polydioxanone, polyesters biodégradables, polyuréthanes, polyacrylates, collagène, fibrinogène.

11. Procédé selon l'une des revendications 1 à 8, **caractérisé en ce que** la matrice tissulaire utilisée est une matrice tissulaire xénogène ou allogénique native ou acellularisée.

12. Procédé selon l'une des revendications 1 à 11, **caractérisé en ce que** les cellules tolérées par le receveur utilisées sont des cellules autologues issues du receveur greffé ou implanté.

13. Procédé selon l'une des revendications 1 à 11, **caractérisé en ce que** les cellules tolérées par le receveur utilisées sont des cellules allogéniques ou des cellules allogéniques modifiées génétiquement choisies du fait qu'elles sont tolérées par le receveur.
